# EUROPEAN PATENT SPECIFICATION

(11) **EP 3 110 964 B1**
(45) Date of publication and mention of the grant of the patent: **30.12.2020**
(21) Application number: 14884109.1
(22) Date of filing: 27.02.2014
(51) Int. Cl.: C12Q 1/28, A01K 29/00, B01J 20/24, B01J 20/28, C12Q 1/26, G01N 33/52, A01K 1/015

(54) **CHROMOGENIC ABSORBENT MATERIAL FOR ANIMAL LITTER**
CHROMOGENES SAUGFÄHIGES MATERIAL FÜR TIERSTREU
MATÉRIAU ABSORBANT CHROMOGÈNE POUR LITIÈRE ANIMALE

(43) Date of publication of application: 04.01.2017
(73) Proprietor: 7905122 Canada Inc., Boucherville QC J4B 2X6 (CA)
(72) Inventor: JOLLEZ, Paul, Sherbrooke, Québec J1J 1G9 (CA)
(74) Representative: Gevers & Orès
(86) International application number: PCT/CA2014/050140
(87) International publication number: WO 2015/127528

(56) References cited:
- WO-A1-2010/133001
- CA-A1- 2 607 753
- DE-U1-202013 005 052
- FR-A1- 2 303 290
- US-A- 4 340 669
- US-A1- 2003 170 905
- US-B1- 6 376 252
- US-B2- 7 533 630

## Description

### FIELD

The technical field relates to animal litter including chromogenic absorbent material for detecting animal diseases.

### BACKGROUND

Domestic animals such as cats are susceptible to various diseases, ailments and conditions, which are not only arduous and painful for the animal itself but also a source of concern and stress for animal owners. While animal owners nurture, watch over and bestow affection on their pets, they must balance this attention with other responsibilities. Convenience is thus an important factor when taking care of a domestic animal. While owners may be devoted and considerate to their pets, they may lack the sophistication to diagnose animal diseases, ailments and conditions. Convenient, simple and effective means to inform pet owners of the presence of diseases, such as urinary infections, are desired so that appropriate steps can be taken to reverse, mitigate or avoid serious illness in the animal.

For example, feline urinary tract disease can be a serious condition for cats. In feline urinary tract disease, crystals of magnesium ammonium phosphate can precipitate in the cat's urinary tract and cause obstruction. If untreated, the obstruction can lead to intense pain and can often be fatal within days. In some cases, upon observing feline urinary tract disease symptoms - such as bloody urine and urination discomfort and straining - cat owners often consult their veterinarian who may be able to provide treatments, which may be expensive. However, many cats with feline urinary tract disease do not show any obvious symptoms, which is why this disease has been referred to as a "silent killer". Another example of a serious condition for cats is diabetes. Diabetes strikes about 1 in 400 cats and has become increasingly common. Symptoms of diabetes in cats are similar to those in humans, and about 80% to 95 % of diabetic cats experience something similar to type-2 diabetes in humans. Cats suffering of diabetes usually become severely insulin-dependent by the time symptoms are diagnosed. In cats suffering from type-2 diabetes, early treatment can sometimes lead to diabetic remission, in which the cat no longer needs injected insulin. If left untreated, the condition leads to increasingly weak cats, malnutrition, ketoacidosis and/or dehydration, and eventually death.

Early detection of diseases such as feline urinary tract disease or diabetes is therefore of paramount importance in facilitating treatment, lessening the likelihood of severe complications or aggravations, and reducing the cost of treatment.

Some methods of early detection are known. Early detection may be possible by occult blood testing, allowing animal owners to treat the problems of urinary tract disease or diabetes by changing the animals' diets or by seeking the help of a veterinarian. However, some known occult blood or glucose testing techniques present various disadvantages concerning the complexity and inconvenience of the tests. For instance, animals will often resist urine sample gathering.

It is known to use diagnostic agents, incorporated into test strips, beads or particles, for detection purposes. Usually, such test strips consist of an absorbent carrier made from fibrous or non-woven material, in the simplest case filter paper, which is coated or impregnated with the detection reagents. Components of the detection reagent may be a chromogenic compound as an indicator, an oxidizing agent such as a hydroperoxide as an oxidizer of the indicator. The oxidizing agent is sometimes also called a sensitizer or an accelerator. Standard additional components are, apart from a surface-active agent (wetting agent), thickening agents which prevent the bleeding of the wetted test field, pigments, complex-forming agents and/or other stabilizers for the chromogen and/or the hydroperoxide.

Similarly, various analytical methods are presently available for detecting the presence of "peroxidatively active substances" in samples such as urine, fecal suspensions, and gastrointestinal contents. According to US 4,460,684, hemoglobin and its derivatives are typical of such peroxidatively active substances because they behave in a manner similar to the enzyme peroxidase. Such substances are also referred to as pseudoperoxidases. Peroxidatively active substances are enzyme-like in that they catalyze the redox reaction between peroxides and benzidine, o-tolidine, 3,3',5,5'-tetramethylbenzidine, 2,7-diaminofluorene or similar benzidine-type indicator substances, thereby producing a detectable response such as a color change. For example, most methods for determining the presence of occult blood in test samples rely on this pseudoperoxidatic activity. A benzidine-type indicator responds in the presence of hydroperoxide and/or peroxidase by changing its light absorptive capability.

Providing a reliable occult blood or glucose detection system in animal litter itself also has many problems and challenges. For example, the test indicator material should be stable when exposed to a wide variety of ambient conditions, be they dry or humid, and over a wide range of temperatures. Such stability is quite often difficult to achieve.

A further problem with many known test indicators is that pet owners are insufficiently observant or sophisticated to appreciate the positive indication, such as a color change, before the indicator decays. Many known indicators do not stay at the changed color for a sufficient period of time to allow pet owners to reliably recognize the indicated health issue.

An additional problem with various detection reagents mixed with animal litter is that the test reagents give off sufficient scent such that cats, which have an extraordinary sense of smell, recognize the odor change in their litter and thus tend to shy away from the litter. As will be appreciated, this not only defeats the purpose of a convenient detector but can also cause unwanted excretory mishaps. Thus, test reagents with significant, offensive or upsetting odors - both to the user and the cat - have many disadvantages.

A further problem with known detection reagents is poor shelf life stability, particularly if combined with an animal litter for storage as a single mixture. Poor stability leads to disadvantages in the ability to store, transport, display, purchase and use the detection-litter combination.

Detection materials that are merely coated over the surface of a carrier material also have various disadvantages that may relate to poor shelf-life stability, low in-use stability and lifetime, and insufficient color change visibility.

Known materials and methods for detection of feline urinary tract disease or diabetes have involved one or more of the above deficiencies.

Some detection methods are disclosed in WO 2010133001 (Jollez et al.) which describes a chromogenic composite material for use with animal litter. The composite material can include an absorptive polymer material; clay; a chromogenic indicator; and an oxidizing agent that is available and responsive to peroxidase or pseudoperoxidase activity in the feline urine to activate the chromogenic indicator. The chromogenic indicator may be 3,3',5,5'-tetramethylbenzidine, also referred to as TMB.

Despite the developments in detection methods for animal excretion tract disease, there is still a need for an improved technology.

### SUMMARY

A chromogenic absorbent material is described herein for detecting substances in animal excretions. The chromogenic absorbent material according to the invention comprises:
an oxidizing agent responsive to peroxidatic/pseudoperoxidatic activity in an animal excretion to provide oxidizing activity; a chromogenic indicator being chromogenically responsive to the oxidizing activity of the oxidizing agent; and an absorptive material which is porous, for absorbing the animal excretion, the absorptive material comprising a water-absorbing polysaccharide,
wherein the chromogenic absorbent material is a porous material having an effective porosity of about 0.5 mL/g to about 2.0 mL/g, wherein the chromogenic absorbent material has a free swelling capacity greater than about 900%, and wherein the chromogenic absorbent material is provided with pores having an equivalent diameter greater than about 20 µm or of about 20 µm to 40 µm.

It is noted that the chromogenic absorbent material according to the invention always exhibits the effective porosity, free swelling capacity and equivalent pore diameter as defined above, even if it is not laid out explicitly for each implementation disclosed in the following.

In some implementations, there is provided a chromogenic absorbent material for an animal litter, comprising:
an oxidizing agent responsive to peroxidatic/pseudoperoxidatic activity in an animal excretion to provide oxidizing activity;
a chromogenic indicator being chromogenically responsive to the oxidizing activity of the oxidizing agent; and
an absorptive material which is porous, for absorbing the animal excretion, the absorptive material comprising:
   a water-absorbing polysaccharide providing absorptive properties to the chromogenic absorbent material; and
   a second polysaccharide providing structural integrity to the chromogenic absorbent material.

In some implementations, the second polysaccharide comprises a crystalline polysaccharide.

In some implementations, the crystalline polysaccharide comprises cellulose, a cellulose derivative or mixtures thereof.

In some implementations, the cellulose comprises microcrystalline cellulose (MCC), nanocrystalline cellulose (NCC), or a mixture thereof.

In some implementations, the absorptive material comprises:
about 35 wt.% to about 65 wt.% of the water-absorbing polysaccharide; and
about 35 wt.% to about 65 wt.% of the second polysaccharide.

In some implementations, the absorptive material comprises:
about 45 wt.% to about 55 wt.% of the water-absorbing polysaccharide; and
about 45 wt.% to about 55 wt.% of the second polysaccharide.

In some implementations, there is provided a chromogenic absorbent material for an animal litter, comprising:
an oxidizing agent responsive to peroxidatic/pseudoperoxidatic activity in an animal excretion to provide oxidizing activity;
a chromogenic indicator being chromogenically responsive to the oxidizing activity of the oxidizing agent; and
an absorptive material which is porous, for absorbing the animal excretion, the absorptive material comprising a water-absorbing polysaccharide,
wherein the chromogenic absorbent material has a density of about 0.20 g/cm³ to about 0.39 g/cm³.

In some implementations, the density of the chromogenic absorbent material is about 0.25 g/cm³ to about 0.35 g/cm³.

In some implementations, the density of the chromogenic absorbent material is about 0.30 g/cm³ to about 0.35 g/cm³.

According to the invention, the chromogenic absorbent material is a porous material having an effective porosity of about 0.5 mL/g to about 2.0 mL/g.

In some implementations, the effective porosity is of about 0.6 mL/g to about 1.5 mL/g.

In some implementations, the effective porosity is of about 0.8 mL/g to about 1.2 mL/g.

In some implementations, the effective porosity is of about 0.9 mL/g to about 1.1 mL/g.

According to the invention, the chromogenic absorbent material is provided with pores having an equivalent diameter greater than about 20 µm or is provided with pores having an equivalent diameter of about 20 µm to about 40 µm.

In some implementations, the chromogenic absorbent material is provided with pores having an equivalent diameter of about 20 µm to about 30 µm.

According to the invention, the chromogenic absorbent material has a free swelling capacity greater than about 900%.

In some implementations, the chromogenic absorbent material has a free swelling capacity greater than about 1000%.

In some implementations, there is provided a chromogenic absorbent material for an animal litter, comprising:
an oxidizing agent responsive to peroxidatic/pseudoperoxidatic activity in an animal excretion to provide oxidizing activity;
a chromogenic indicator being chromogenically responsive to the oxidizing activity of the oxidizing agent; and
an absorptive material which is porous, for absorbing the animal excretion, the absorptive material comprising:
   a water-absorbing polysaccharide providing absorptive properties to the chromogenic absorbent material; and
   a superabsorbent polymer (SAP).

In some implementations, the absorptive material comprises up to about 3 wt.% of the SAP.

In some implementations, the absorptive material comprises about 1 wt.% to about 3 wt.% of the SAP.

In some implementations, the absorptive material comprises about 1 wt.% to about 2 wt.% of the SAP.

In some implementations, the SAP comprises at least one of a poly(acrylic acid) and a poly(methacrylic acid), or a salt thereof.

In some implementations, there is provided a chromogenic absorbent material for an animal litter, comprising:
an oxidizing agent responsive to peroxidatic/pseudoperoxidatic activity in an animal excretion to provide oxidizing activity;
a chromogenic indicator being chromogenically responsive to the oxidizing activity of the oxidizing agent; and
an absorptive material which is porous, for absorbing the animal excretion, the absorptive material comprising:
   a water-absorbing polysaccharide providing absorptive properties to the chromogenic absorbent material; and
   a second polysaccharide providing structural integrity to the chromogenic absorbent material,
wherein the chromogenic absorbent material is a porous material having:
   an effective porosity of about 0.5 mL/g to about 2.0 mL/g; and
   a density of about 0.20 g/cm³ to about 0.39 g/cm³.

In some implementations, the water-absorbing polysaccharide comprises a starch, a modified starch, a cellulose derivative or a gelling polysaccharide, or a mixture thereof.

In some implementations, the water-absorbing polysaccharide comprises pregelatinized starch.

In some implementations, the cellulose derivative comprises a cellulose ester or a cellulose ether, or a mixture thereof.

In some implementations, the cellulose derivative comprises carboxymethyl cellulose (CMC).

In some implementations, the gelling polysaccharide comprises agar-agar, guar or xanthan, or a mixture thereof.

In some implementations, the oxidizing agent comprises a hydroperoxide or a hydroperoxide precursor, or a combination thereof.

In some implementations, the hydroperoxide comprises hydrogen peroxide, cumene hydroperoxide or diisopropylbenzene dihydroperoxide, or a combination thereof.

In some implementations, the oxidizing agent and the chromogenic indicator are distributed within the absorptive material.

In some implementations, the chromogenic indicator comprises a benzidine-type compound.

In some implementations, the benzidine-type compound comprises 3,3',5,5'-tetramethylbenzidine.

In some implementations, the chromogenic absorbent material further comprises a buffering agent, a stabilizer, a metal scavenger agent or a color enhancer or a combination thereof.

In some implementations, the color enhancer comprises 6-methoxyquinoline, lepidin, phenol derivatives, nitrobenzene, N-methylpyrrolidone or ethylene carbonate or a combination thereof.

In some implementations, the buffering agent comprises citrate, sodium citrate, phosphate or acetate or a combination thereof.

In some implementations, the stabilizer comprises ammonium molybdate, polyethylene glycol, polyvinylpyrrolidone, polyethylene oxide or derivatives thereof or a combination thereof.

In some implementations, the metal-scavenger agent comprises ethylenediaminetetraacetic acid (EDTA) or EDTA sodium salt or a combination thereof.

In some implementations, the chromogenic indicator is responsive to the oxidizing agent by turning blue in presence of the peroxidatic/pseudoperoxidatic activity in the animal excretions.

In some implementations, the chromogenic absorbent material turns to blue in presence of the peroxidatic/pseudoperoxidatic activity after a contact time with the animal excretion between about 10 seconds and about 30 min.

In some implementations, the chromogenic absorbent material turns to blue in presence of the peroxidatic/pseudoperoxidatic activity after a contact time with the animal excretion between about 10 seconds and about 1 min.

In some implementations, there is provided a chromogenic absorbent material for an animal litter, comprising:
a first catalytic compound for in situ generation of an oxidizing agent responsive to peroxidatic/pseudoperoxidatic activity in an animal excretion, the oxidizing agent providing oxidizing activity;
a chromogenic indicator being chromogenically responsive to the oxidizing activity of the oxidizing agent;
a second catalytic compound for catalyzing the oxidation of the chromogenic indicator upon in situ generation of the oxidizing agent; and
an absorptive material which is porous, for absorbing the animal excretion, the absorptive material comprising:
   a water-absorbing polysaccharide providing absorptive properties to the chromogenic absorbent material; and
   a second polysaccharide providing structural integrity to the chromogenic absorbent material.

In some implementations, there is provided a chromogenic absorbent material for an animal litter, comprising:
a first catalytic compound for in situ generation of an oxidizing agent responsive to peroxidatic/pseudoperoxidatic activity in an animal excretion, the oxidizing agent providing oxidizing activity;
a chromogenic indicator being chromogenically responsive to the oxidizing activity of the oxidizing agent;
a second catalytic compound for catalyzing the oxidation of the chromogenic indicator upon in situ generation of the oxidizing agent; and an absorptive material for absorbing the animal excretion, the absorptive material comprising a water-absorbing polysaccharide,
wherein the chromogenic absorbent material has a density of about 0.20 g/cm³ to about 0.39 g/cm³.

In some implementations, there is provided a chromogenic absorbent material for an animal litter, comprising:
a first catalytic compound for in situ generation of an oxidizing agent responsive to peroxidatic/pseudoperoxidatic activity in an animal excretion, the oxidizing agent providing oxidizing activity;
a chromogenic indicator being chromogenically responsive to the oxidizing activity of the oxidizing agent;
a second catalytic compound for catalyzing the oxidation of the chromogenic indicator upon in situ generation of the oxidizing agent; and an absorptive material for absorbing the animal excretion, the absorptive material comprising a water-absorbing polysaccharide,
wherein the chromogenic absorbent material is a porous material having an effective porosity of about 0.5 mL/g to about 2.0 mL/g.

In some implementations, there is provided a chromogenic absorbent material for an animal litter, comprising:
a first catalytic compound for in situ generation of an oxidizing agent responsive to peroxidatic/pseudoperoxidatic activity in an animal excretion, the oxidizing agent providing oxidizing activity;
a chromogenic indicator being chromogenically responsive to the oxidizing activity of the oxidizing agent;
a second catalytic compound for catalyzing the oxidation of the chromogenic indicator upon in situ generation of the oxidizing agent; and
an absorptive material which is porous, for absorbing the animal excretion, the absorptive material comprising:
   a water-absorbing polysaccharide providing absorptive properties to the chromogenic absorbent material; and
   a superabsorbent polymer (SAP).

In some implementations, there is provided a chromogenic absorbent material for an animal litter, comprising:
a first catalytic compound for in situ generation of an oxidizing agent responsive to peroxidatic/pseudoperoxidatic activity in an animal excretion, the oxidizing agent providing oxidizing activity;
a chromogenic indicator being chromogenically responsive to the oxidizing activity of the oxidizing agent;
a second catalytic compound for catalyzing the oxidation of the chromogenic indicator upon in situ generation of the oxidizing agent; and
an absorptive material which is porous, for absorbing the animal excretion, the absorptive material comprising:
   a water-absorbing polysaccharide providing absorptive properties to the chromogenic absorbent material; and
   a second polysaccharide providing structural integrity to the chromogenic absorbent material,
wherein the chromogenic absorbent material is a porous material having:
   an effective porosity of about 0.5 mL/g to about 2.0 mL/g; and
   a density of about 0.20 g/cm³ to about 0.39 g/cm³.

In some implementations, the water-absorbing polysaccharide comprises a cellulose derivative or a gelling polysaccharide, or a mixture thereof.

In some implementations, the first catalytic compound comprises an oxido-reductase enzyme.

In some implementations, the oxido-reductase comprises glucose oxidase (GOx).

In some implementations, the oxidizing agent generated in situ is hydrogen peroxide.

In some implementations, the second catalytic compound comprises a peroxidase, a pseudoperoxidase, or a mixture thereof.

In some implementations, the peroxidase comprises horseradish peroxidase (HRP).

In some implementations, the first catalytic compound, the second catalytic compound and the chromogenic indicator are distributed within the absorptive material.

Further disclosed, but not according to the invention is a chromogenic absorbent material for detecting a detectable substance in an animal excretion, the chromogenic absorbent material comprising:
a trigger agent;
a chromogenic indicator oxidizable into a colored and/or fluorescent substance in the presence of the trigger agent and the detectable substance; and
an absorptive material which is porous, for absorbing the animal excretion, the absorptive material comprising:
   a water-absorbing polysaccharide providing absorptive properties to the chromogenic absorbent material; and
   a second polysaccharide providing structural integrity to the chromogenic absorbent material.

Further disclosed, but not according to the invention is a chromogenic absorbent material for detecting a detectable substance in an animal excretion, the chromogenic absorbent material comprising:
a trigger agent;
a chromogenic indicator oxidizable into a colored and/or fluorescent substance in the presence of the trigger agent and the detectable substance; and
an absorptive material which is porous, for absorbing the animal excretion, the absorptive material comprising:
   a water-absorbing polysaccharide providing absorptive properties to the
   chromogenic absorbent material,
wherein the chromogenic absorbent material has a density of about 0.20 g/cm³ to about 0.39 g/cm³.

Further disclosed, but not according to the invention is a chromogenic absorbent material for detecting a detectable substance in an animal excretion, the chromogenic absorbent material comprising:
a trigger agent;
a chromogenic indicator oxidizable into a colored and/or fluorescent substance in the presence of the trigger agent and the detectable substance; and
an absorptive material which is porous, for absorbing the animal excretion, the absorptive material comprising:
   a water-absorbing polysaccharide providing absorptive properties to the
   chromogenic absorbent material,
wherein the chromogenic absorbent material is a porous material having an effective porosity of about 0.5 mL/g to about 2.0 mL/g.

Further disclosed, but not according to the invention is a chromogenic absorbent material for detecting a detectable substance in an animal excretion, the chromogenic absorbent material comprising:
a trigger agent;
a chromogenic indicator oxidizable into a colored and/or fluorescent substance in the presence of the trigger agent and the detectable substance; and
an absorptive material which is porous, for absorbing the animal excretion, the absorptive material comprising:
   a water-absorbing polysaccharide providing absorptive properties to the chromogenic absorbent material; and
   a superabsorbent polymer (SAP).

Further disclosed, but not according to the invention is the use of the chromogenic absorbent material as chromogenic particles in combination with animal litter.

In some implementations, the animal litter comprises clay based particles, cellulosic particles, perlite based particles, silica based particles, corn based particles, paper based particles or wheat based particles or a combination thereof.

In some implementations, the clay based particles comprise montmorillonite.

In some implementations, the clay based particles comprise bentonite.

In some implementations, the chromogenic absorbent material is used for detecting blood in animal excretions.

In some implementations, the chromogenic absorbent material is used for detecting glucose in animal excretions.

In some implementations, the chromogenic particles are substantially evenly distributed on a top surface of the animal litter.

In some implementations, the chromogenic particles are substantially evenly distributed within the animal litter.

In some implementations, the chromogenic particles comprise pellets, granules, disks, squares according to their process of manufacture.

Further disclosed, but not according to the invention is a chromogenic absorbent material for detecting a detectable substance in an animal excretion, the chromogenic absorbent material comprising:
a trigger agent;
a chromogenic indicator convertable into a colored and/or fluorescent substance in the presence of the trigger agent and the detectable substance; and
an absorptive material for absorbing the animal excretion, the absorptive material having a porous and a block-shaped microstructure.

### BRIEF DESCRIPTION OF THE DRAWINGS

Fig. 1 is a scheme of the reaction pathway taking place in the particles of chromogenic absorbent material for the detection of blood in animal excretions.
Fig. 2 is a scheme of the reaction pathway taking place in the particles of chromogenic absorbent material for the detection of glucose in animal excretions.
Fig. 3A shows photographs of six samples of particles of chromogenic absorbent materials after 30 minutes, 2 hours and 18 hours of contact with a diluted blood solution.
Fig. 3B shows photographs of six samples of particles of chromogenic absorbent materials including 1% of superabsorbent polymer after 30 minutes, 2 hours and 18 hours of contact with a diluted blood solution.
Fig. 3C shows photographs of six samples of particles of chromogenic absorbent materials including 2% of superabsorbent polymer after 30 minutes, 2 hours and 18 hours of contact with a diluted blood solution.
Fig. 3D shows photographs of six samples of particles of chromogenic absorbent materials including 3% of superabsorbent polymer after 30 minutes, 2 hours and 18 hours of contact with a diluted blood solution.
Fig. 4 shows photographs of three samples of particles of chromogenic absorbent materials including after 6h30 and 22 hours of contact with a diluted blood solution.
Fig. 5 shows photographs of samples of particles of chromogenic absorbent materials including after 1 minute and 10 minutes of contact with glucose solutions of different concentrations.
Fig. 6A is a x200 scanning electron micrograph showing the surface of an extruded starch particle (comparative Figure).
Fig. 6B is a x200 scanning electron micrograph showing the surface of an extruded starch particle in which gas was injected during extrusion (comparative figure).
Fig. 6C is a x200 scanning electron micrograph showing the surface of a particle of chromogenic absorbent material in which the absorptive material includes 50% PGS and 50% MCC.
Fig. 6D is a x200 scanning electron micrograph showing the surface of a particle of pressed cellulose (comparative figure).
Fig. 7A is a x200 scanning electron micrograph showing a cross section of an extruded starch particle, obtained by freeze-fracture (comparative Figure).
Fig. 7B is a x200 scanning electron micrograph showing a cross section of an extruded starch particle in which gas was injected during extrusion. The cross section is obtained by freeze-fracture (comparative figure).
Fig. 7C is a x200 scanning electron micrograph showing a cross section of a particle of chromogenic absorbent material in which the absorptive material includes 50% PGS and 50% MCC.
Fig. 8A is a x400 scanning electron micrograph showing a cross section of an extruded starch particle, obtained by freeze-fracture (comparative Figure).
Fig. 8B is a x400 scanning electron micrograph showing a cross section of an extruded starch particle in which gas was injected during extrusion. The cross section is obtained by freeze-fracture (comparative figure).
Fig. 8C is a x400 scanning electron micrograph showing a cross section of a particle of chromogenic absorbent material in which the absorptive material includes 50% PGS and 50% MCC.
Fig. 9 shows photographs of extruded starch particles (9A, comparative), extruded starch particles in which gas was injected during extrusion (9B, comparative), particles of chromogenic absorbent material in which the absorptive material includes 50% PGS and 50% MCC (9C), and particles of pressed cellulose (9D, comparative).

### DETAILED DESCRIPTION

A chromogenic absorbent material may include an oxidizing agent, a chromogenic indicator and an absorptive material, for detecting disease features when contacted with animal excretions. The absorptive material includes a water-absorbing polysaccharide and optionally a second crystalline polysaccharide and/or a superabsorbent polymer, and may also have high-porosity and low-density properties.

In some implementations, the chromogenic absorbent material is provided for detecting blood or glucose in excretions. More particularly, the chromogenic absorbent material may be used in connection with an animal litter. Processes for making chromogenic absorbent materials are also described.

It should be understood that excretion refers to any matter excreted by an animal, such as urine or fecal matter. The chromogenic absorbent material may be used in any domestic animal litter including cat litter, dog litter and rodent litter. It may also be used for horse litter, cow litter or any other livestock litter. However, various implementations of the chromogenic absorbent material are not limited to detecting blood or glucose in animal excretions and may be used to detect blood or glucose in human excretions, for example.

Particles of the chromogenic absorbent material may be dispersed within the animal litter or at the surface of the animal litter. The animal litter may include clay based particles, cellulosic particles, perlite based particles, silica based particles, corn based particles, paper based particles, wheat based particles or other organic-based litter particles, or a combination thereof. For example and without being limitative, clay based particles may include bentonite and/or montmorillonite.

In some implementations, the particles of chromogenic absorbent material include: an oxidizing agent responsive to peroxidatic/pseudoperoxidatic activity in an animal excretion to provide oxidizing activity, or a first catalytic compound generating the oxidizing agent in situ; a chromogenic indicator being chromogenically responsive to the oxidizing activity of the oxidizing agent; and an absorptive material for absorbing the animal excretion, the absorptive material including a water-absorbing polysaccharide providing absorptive properties to the chromogenic absorbent material.

In some implementations, the oxidizing agent and/or the first catalytic compound, and the chromogenic indicator are distributed on at least an exterior surface of the absorptive material. In some implementations, the oxidizing agent and/or the first catalytic compound, and the chromogenic indicator are distributed within the absorptive material.

It should be understood that the expression "peroxidatic activity" refers to the ability of catalytic compounds to drive the reaction between hydroperoxides and colorless chromogenic electron donors which become fluorescent or visibly colored after oxidation. It should be understood that the expression "pseudoperoxidatic activity" refers to the ability of a peroxidase or a non-peroxidase catalytic compound to drive the reaction between hydroperoxidases and colorless chromogenic electron donors which become fluorescent or visibly colored after oxidation. Certain transition metals and their ions and hemoproteins are known to have pseudoperoxidatic activity. Basophils, neutrophils, eosinophils and mast cells synthesize endogenous peroxidase which can be visualized at the ultrastructural level in the secretory apparatus of immature cells. Red blood cells and hematin containing compounds have iron as part of their heme groups, which can catalyze the oxidation of chromogenic electron donors. This pseudoperoxidatic activity can be inhibited with strong H₂O₂ solutions, sodium azide and methanol-H₂O₂ solutions.

It should be understood that "particle" refers to any pellet, granule or piece of various shapes. Optionally, the particles may generally have a circular cross-section with an average diameter ranging from 2.5 mm to 10mm. Optionally, the particles may generally have a square or rectangular cross-section with an average length ranging from 5 mm to 20 mm. Optionally, the particles may have a top surface ranging from 19 mm² to 400 mm² and a thickness ranging from 1 to 10 mm. The shape(s) of the particles may be conferred by their process of manufacture.

The oxidizing agent is reactive to peroxidatic/pseudoperoxidatic activity and is able to oxidize the chromogenic indicator in the presence of a peroxidase or a pseudoperoxidase. For example, the peroxidase can be horseradish peroxidase. For example, the pseudoperoxidase can be haemoglobin present in blood. In an optional aspect, the oxidizing agent includes a hydroperoxide.

It should be understood that "hydroperoxide" refers to compounds of the general formula ROOH, wherein the R group is an aryl, alkyl or acyl group (organic hydroperoxide), or a hydrogen atom (hydrogen peroxide). For example and without being limitative, the hydroperoxide can be cumehe hydroperoxide (CHP), diisopropylbenzene dihydroperoxide or hydrogen peroxide, or a mixture thereof. Hydroperoxides are suitable for the detection of peroxidatic/pseudoperoxidatic activity.

In some implementations, the oxidizing agent may be a hydroperoxide precursor such as sodium percarbonate. Sodium percarbonate is a chemical adduct of sodium carbonate and hydrogen peroxide. The formula of sodium percarbonate is 2Na₂CO₃·3H₂O₂. Sodium percarbonate decomposes to sodium carbonate and hydrogen peroxide, for example upon contact with water.

In some implementations, the oxidizing agent is not initially added to the chromogenic absorbent material, but is generated in situ by a first catalytic compound present in the chromogenic absorbent material. It should be understood that "generated in situ" means that the oxidizing agent is directly synthesized in the chromogenic absorbent material from a precursor. For example, the first catalytic compound may be an enzyme such as an oxido-reductase. For example, the first catalytic compound may be glucose oxidase (GOx). Optionally, the precursor may be oxygen (O₂), which can be reduced to hydrogen peroxide in the presence of glucose oxidase. In an optional aspect, the reduction of the precursor to the oxidizing agent can take place in the presence of a saccharide or polysaccharide which can be oxidized by the first catalytic compound.

In some implementations, the oxidizing activity of the oxidizing agent is triggered by the presence of peroxidatic/pseudoperoxidatic activity in excretions. The oxidizing agent therefore oxidizes the chromogenic indicator which then changes of color. More particularly, the chromogenic indicator is an electron donor, i.e. a reducing agent that changes color upon losing an electron.

In some implementations, the chromogenic indicator is a benzidine-type compound, i.e. a compound as shown in formula I:

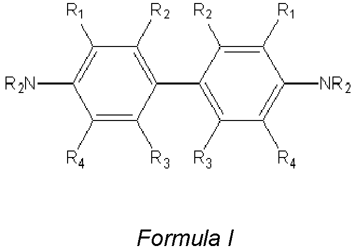

In Formula I, groups R₁, R₂, R₃ and R₄ may be the same or different and may be hydrogen, halogen, a lower alkyl or alkoxy group containing 1 to 4 carbon atoms, a (C₁-C₄)-dialkylamino group, an acetylamino group, a nitro group or an aromatic group which may be substituted.

Optionally, the chromogenic indicator may be a compound as shown in Formula II:

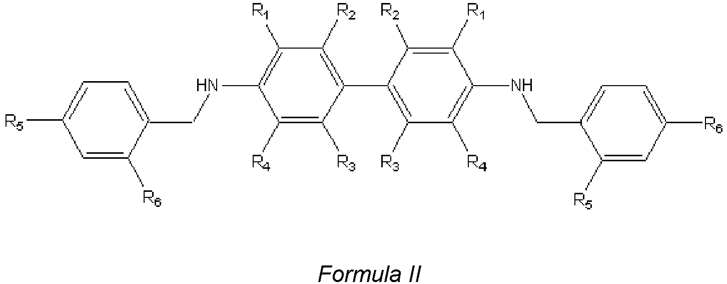

In Formula II, groups R₁, R₂, R₃ and R₄ may be the same or different and represent hydrogen, halogen, and a lower alkyl or alkoxy group containing 1 to 4 carbon atoms, a (C₁-C₄)-dialkylamino group, an acetylamino group, a nitro group or an aromatic group which may be substituted; R₅ and R₆ are the same or different and represent watersoluble groups as hydroxyl groupl, amino group, acidic group, disulfonyl group, ether group, halogen, and a lower alkyl or alkoxy group containing 1 to 4 carbon atoms, a (C₁-C₄)-dialkylamino group, an acetylamino group or a nitro group.

Thus, a water soluble benzidine-type chromogenic indicator of Formula II, responds in the presence of hydroperoxide and peroxidase by changing its light absorptive capability, which is due to the chemical transformation to the compound shown in Formula III:

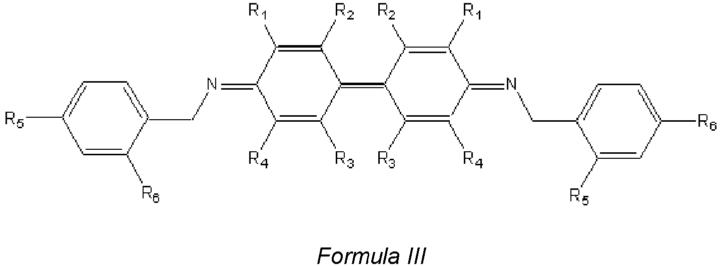

It is understood that several different types of benzidine chromogenic indicators may be used.

Optionally, the chromogenic indicator may be 3,3',5,5'-tetramethylbenzidine (TMB). TMB is a colorless agent which turns blue upon oxidation. The peroxidase and/or pseudoperoxidase catalyze the oxidation of TMB by the oxidizing agent (hydroperoxide) according to the following oxidation reaction.

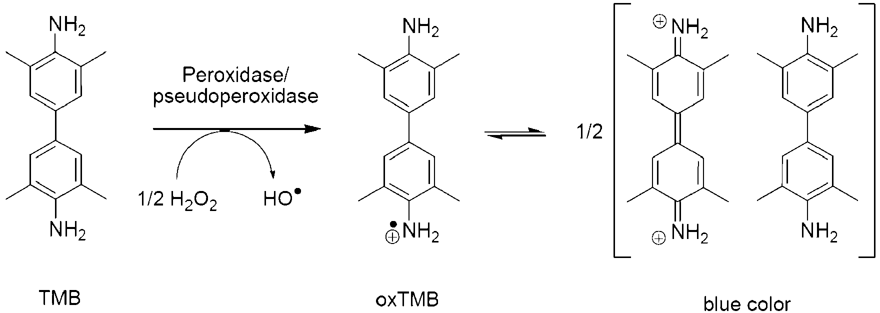

The absorptive material includes a water-absorbing polysaccharide providing absorptive properties to the chromogenic absorbent material. In some implementations, the water-absorbing polysaccharide may be a starch, a modified starch, amylopectin, amylose, modified amylose, a cellulose derivative, a gelling polysaccharide or a mixture thereof. Non-limiting examples of starches and modified starches are starch granules, pregelatinized starch, waxy starches, anionic starches, cationic starches, fractionated starches, cross-linked starches or mixtures thereof. Such starches may be obtained from many sources, including but not limited to wheat, maize, buckwheat, potato, cassaya, sorghum, millet, oat, arrowroot, barley, beans, peas, rice, rye, and mixtures thereof. Non-limiting examples of cellulose derivatives are cellulose esters and cellulose ethers, or a mixture thereof. A non-limiting example of a cellulose ether is carboxymethyl cellulose (CMC). Non-limiting examples of gelling polysaccharides are agar-agar, guar and xanthan, or a mixture thereof.

Optionally, the water-absorbing polysaccharide can be a glass-like polysaccharide. Glass-like polysaccharides are substantially amorphous polysaccharides and include glass-like characteristics. Glass-like polysaccharides substantially lack an organized crystalline pattern. Glass-like polysaccharides are typically prepared by melting or heating the polysaccharide to a temperature above its glass-transition temperature, followed by cooling to a temperature below its glass transition or melting point temperature. A non-limiting example of a glass-like polysaccharide is pregelatinized starch.

Optionally, the absorptive material further includes a superabsorbent polymer (SAP). Optionally, the absorptive material includes in weight up to about 3 wt.%, or between 1 wt.% and 2.5 wt.% of the SAP. Non-limiting examples of SAP are poly(acrylic acids) and poly(methacrylic acids), salts thereof, or mixtures thereof. A non-limiting example of SAP is sodium polyacrylate, which is an efficient SAP. It should be understood that other types of SAPs may be used, such as superabsorbent starches or other synthetic superabsorbent polymers.

In an optional aspect, each particle of chromogenic absorbent material further includes a second polysaccharide providing structural integrity to the chromogenic absorbent material. By "providing structural integrity", it is meant that the second polysaccharide reduces or prevents the breaking up of the particles of chromogenic absorbent material upon handling or upon contact with an animal excretion. In other words, the second polysaccharide reduces the brittleness of the chromogenic absorbent material while preventing an increase of the softness or pliability of the chromogenic absorbent material. In some scenarios, the second polysaccharide provides sufficient structural integrity so that the particles of the chromogenic absorbent material cannot be easily broken or fractured by hand and are relatively unpliable and rigid solids.

For example, when the absorptive material consists of 100% pregelatinized starch, the particles of chromogenic absorbent material can tend to be soft and pliable and thus not as easily manipulated or deposited onto animal litter without being damaged. Upon contact with animal excretions, such pliable particles can still provide the desired color change and activity, but can be more easily crushed, torn or distorted by the animal.

Optionally, the second polysaccharide includes a crystalline polysaccharide. Examples of crystalline polysaccharides are cellulose, cellulose derivatives or mixtures thereof. In an optional aspect, the cellulose includes microcrystalline cellulose (MCC) or nanocrystalline cellulose (NCC), or a mixture thereof. In an optional aspect, the absorptive material includes in weight: about 35% to about 65%, or about 45% to 55% of the water-absorbing polysaccharide; and about 35% to about 65% or about 45% to about 55% of the second polysaccharide. In an optional aspect, the crystalline polysaccharide is less water-absorbent than the water-absorbing polysaccharide.

In some implementations, the chromogenic absorbent material may turn blue upon contact with excretions containing at least traces of blood (with therefore peroxidase/pseudo-peroxidase activity).

It should be understood that "blue" refers to any shade of blue. The chromogenic absorbent material may need a contact time with excretions sufficient to enable coloration. In an optional aspect, the particles may turn blue after a contact time ranging from about 10 seconds to about 30 min, or from about 10 seconds to about 1 min, depending on the nature of the absorptive material of the particles.

In some implementations, the chromogenic absorbent material may turn to different shades of blue depending on the blood or glucose concentration in excretions. The intensity of the blue shade may be proportional to the blood concentration or glucose concentration in excretions.

In some implementations, the chromogenic composition may further include a colour enhancer. Optionally, it may also include a buffering agent, a stabilizer, a metal scavenger agent or a combination thereof. The colour enhancer may optionally be 6-methoxyquinoline, lepidin, phenol derivatives, nitrobenzene, N-methylpyrrolidone, ethylene carbonate or any combination thereof. The buffering agent may optionally include citrate, sodium citrate, phosphate, acetate or any combination thereof. The stabilizer may optionally be ascorbic acid, ammonium molybdate and derivatives thereof, polyethylene glycol, polyvinylpyrrolidone, polyethylene oxide and derivatives thereof, or combination thereof. The metal-scavenger agent may optionally be EDTA, EDTA sodium salt or any combination thereof.

Not according to the invention, a chromogenic absorbent material is provided for detecting a detectable substance in an animal excretion. The chromogenic absorbent material includes:
a trigger agent responsive to the presence of the detectable substance;
a chromogenic indicator convertable into a chromogenically active substance in the presence of the trigger agent and the detectable substance; and
an absorptive material for absorbing the animal excretion, the absorptive material being porous and including:
   a water-absorbing polysaccharide providing absorptive properties to the chromogenic absorbent material; and
   a second polysaccharide providing structural integrity to the chromogenic absorbent material.

It is understood that the trigger agent may be selected depending on the detectable substance and such that the conversion of the chromogenic indicator takes place and/or is catalyzed only if both the trigger agent and the detectable substance are present. For example, when the detectable substance is a peroxidase or a pseudoperoxidase, the trigger agent may be an oxidizing agent responsive to peroxidatic/pseudoperoxidatic activity in the animal excretion and the conversion of the chromogenic indicator includes oxidation into the chromogenically active substance.

The detectable substance includes a pseudoperoxidase (such as blood which includes haemoglobin), and the trigger agent is a hydroperoxide (such as cumene hydroperoxide) or a hydroperoxide precursor.

The detectable substance is glucose, and the trigger agent is a catalytic system including an oxido-reductase and a peroxidase, or an oxido-reductase and a pseudoperoxidase. For example, the oxido-reductase may be glucose oxidase and the peroxidase may be horseradish peroxidase.

Depending on the absorptive material, the particles of chromogenic absorbent material may have a density of about 0.20 g/cm³ to about 0.39 g/cm³, of about 0.20 g/cm³ to about 0.35 g/cm³, of about 0.25 g/cm³ to about 0.35 g/cm³, or of about 0.30 g/cm³ to about 0.35g/cm³.

Depending on the absorptive material, the chromogenic absorbent material may have a total porosity of about 65% to about 85%, or of about 70% to about 80%. It is understood that the total porosity refers to the fraction of the bulk material volume (V) which is not occupied by solid matter. If the volume of solids is denoted by Vs, and the pore volume as Vpore = V-Vs, the total porosity can be expressed as shown in Equation 1 below. $total porosity = ϕ = \frac{V - Vs}{V} = \frac{Vpore}{V} \left(mL/mL\right)$ Equation 1

The total porosity may for example be measured by: placing a known volume of chromogenic absorbent particles into a container; covering the particles with a liquid; and measuring the volume of liquid needed to cover the particles (Vc). The total porosity is then expressed as the ratio of the volume of added liquid (Vc) to the volume of particles (V).

According to the invention, the particles of chromogenic absorbent material have an effective porosity of about 0.5 mL/g to about 2.0 mL/g, of about 0.6 mL/g to about 1.5 mL/g, of about 0.8 mL/g to about 1.2 mL/g or of about 0.9 mL/g to about 1.1 mL/g. It is understood that the effective porosity (also referred to as connected porosity or true porosity) is defined as the ratio of the connected pore volume to the total bulk volume. The effective porosity is measured by: placing a known mass (m) of chromogenic absorbent particles into a container; covering the particles with a liquid; measuring the volume of liquid needed to cover the particles (Vc); removing the soaked particles from the container; measuring the liquid remaining in the container (Vr); and calculating the volume of liquid absorbed in the chromogenic absorbent particles (Va = Vc - Vr). The effective porosity may then be obtained as shown in Equation 2 below. $effective porosity = {ϕ}_{e} = \frac{Vc - Vr}{m} = \frac{Va}{m} \left(ml/g\right)$ Equation 2

It is to be noted that the effective porosity may also be expressed as the ratio Va/V in mL/mL.

In some implementations, the nature and form of the absorptive material may be selected and modified to allow sufficient internal diffusion and retention of excretions to facilitate the chromogenic indicator response over time. For example, the absorptive material may be modified so as to increase its porosity. The chromogenic indicator may also be homogeneously dispersed throughout the absorptive material according to the preparation method of the chromogenic absorbent material. The chromogenic indicator may be present not only at the exterior surface of a given particle, but also in a neighboring sub-surface region that can be rapidly exposed to excretions that are absorbed into the particle. Additionally, when the absorptive material is glassy or substantially transparent, the presence of the chromogenic indicator in a sub-surface region allows it to be readily visible when a color change occurs and also avoids exposure to the air. In addition, the absorptive material may be provided with certain absorptive properties relative to the environment when in operation. For instance, the absorptive material may be provided to enable faster absorption of excretions compared to the surrounding material, such as surrounding animal litter, to facilitate adequate exposure of the excretions to the active agents in the chromogenic absorptive material. As different animal litters may have different absorptive properties, the absorptive material may be provided in accordance with pre-determined litter absorption properties, e.g. according to a maximum litter absorption rate. For instance, in some implementations, the absorptive material has a higher absorption rate compared to the litter material, and optionally a substantially higher absorption rate. For example, the absorptive material may have an absorption rate about 3 to 10 times higher, or about 5 to 10 times higher than the absorption rate of the litter material.

According to the invention, the chromogenic absorbent material has a free swelling capacity (FSC) greater than about 900%, or greater than about 1000%. The FSC is one type of measurement used for measuring the absorption properties of a material. An FSC measurement is performed by soaking the material to be tested in a liquid to be absorbed (in the present case, water) for a given time and weighing the material after the liquid has been absorbed. In some implementations, the chromogenic absorbent material has a higher FSC than compared to the litter material. For example, the chromogenic absorbent material may have a FSC about 1.5 to 2 times higher than the FSC of the litter material.

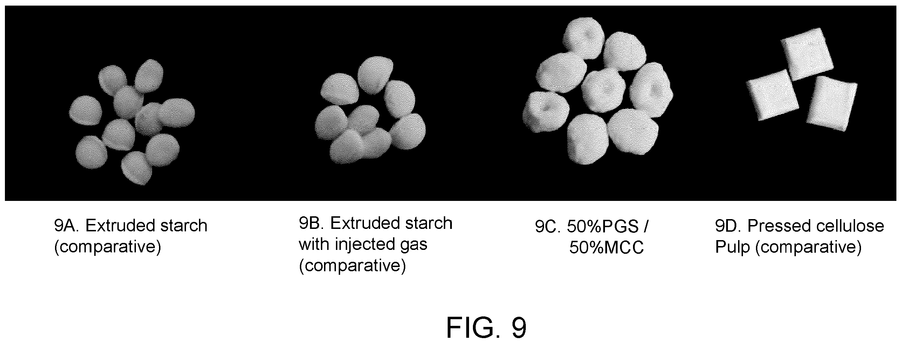

Now referring to Fig. 9, a photograph showing different particles is shown. Particles 9A are extruded starch particles obtained under high shear, without injection of gas during extrusion. Particles 9A were made as a comparative example. Particles 9B are extruded starch particles obtained under high shear, with injection of gas during extrusion, Particles 9B were made as a comparative example. Particles 9D are pressed cellulose pulp particles and were also made as a comparative example. Particles 9C are chromogenic absorbent particles in which the absorptive material includes 50% pregelatinized starch (PGS) and 50% microcrystalline cellulose (MCC). Particles 9C were obtained through a process as described below and correspond to sample 25 as detailed in Example 2.

As can be seen in Fig. 9, particles 9A and 9B are in the form of compact pellets and particles 9D are in the form of pressed, compact squares. Particles 9C of chromogenic absorbent material are in the form of granules having a concave shape on one side and a convex shape on an opposite side. Of course, it is understood that the particles of chromogenic absorbent material may be of different shapes and be manufactured as pellets, granules, disks or squares, according to their process of manufacture.

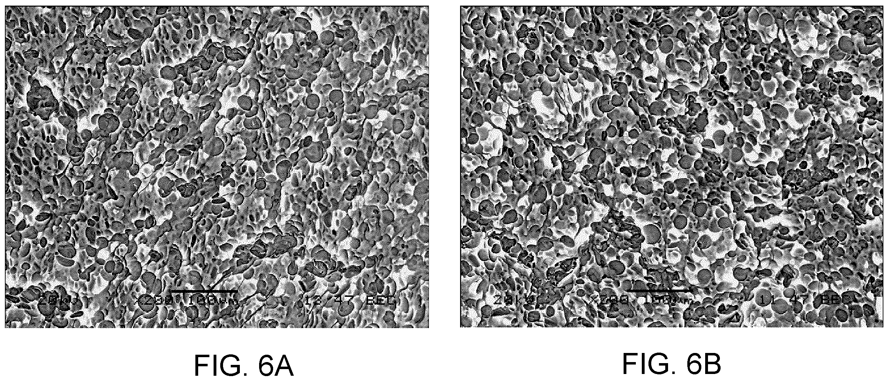

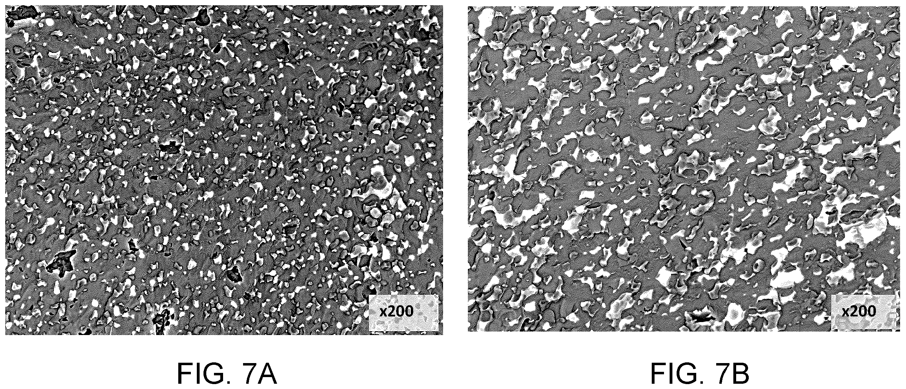

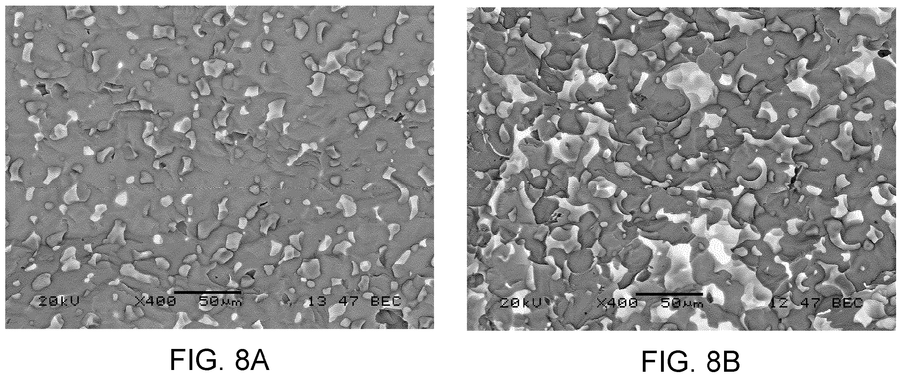

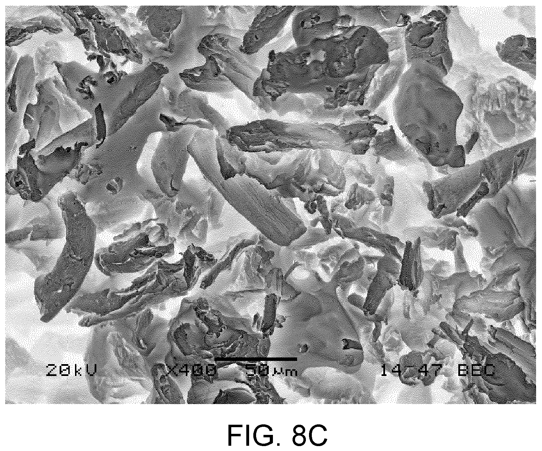

Scanning electron micrographs of the particles of Fig. 9 were obtained in order to compare the morphology of particles 9A, 9B, 9C and 9D. Scanning electron micrographs showing the surface of the particles are shown in Figures 6A to 6D. Scanning electron micrographs showing cross sections of the particles are shown in Figures 7A to 7C and 8A to 8C. The scanning electron microscope used was a MEB JEOL JSM-5900LV™ (low vacuum).

Figs. 6A and 6B (comparative) show the surface of extruded starch particles obtained under high shear, with and without injected gas during extrusion. As can be seen, the surface of the extruded starch includes microscopic starch globules having a size of between about 5 µm and about 30 µm.

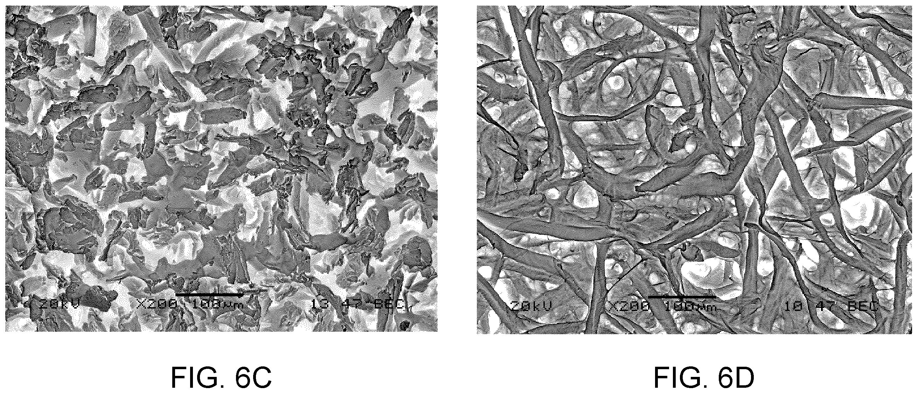

Fig. 6D (comparative) shows the surface of pressed cellulose pulp particles. Elongated cellulose fibers can be seen on the surface. The fibers have a length of between about 100 µm and about 400 µm, and a width of between about 10 µm to about 30 µm.

Fig. 6C shows the surface of chromogenic absorbent particles in which the absorptive material includes 50% pregelatinized starch (PGS) and 50% microcrystalline cellulose (MCC). Microsructures of various shapes can be seen on the micrograph. The microsructures have a length of between about 10 µm to about 100 µm, and a width of between about 10 µm to about 100 µm.

Different microstructure morphologies are apparent for the different particles. The particles of Figs. 6A and 6B mainly include a smooth globular microstructure, the particles of Fig. 6D mainly includes generally smooth filamentous microstructure, while the particles of Fig. 6C mainly include a rough, irregular, block-shaped microstructure.

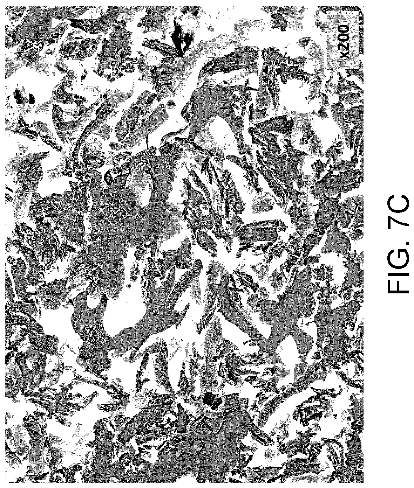

The pore structure of the particles was also studied. Cross sections of the particles of chromogenic absorbent material were observed by scanning electron microscopy, as can be seen in Fig. 7C, and as detailed in Example 6. The cross sections were obtained by freeze-fracture under liquid nitrogen and observed by SEM to determine the pore density and equivalent diameter of the pores. It is understood that "pore density" refers to the proportion of the surface which is not covered by solid material (i.e., the ratio of the pore surface to the total surface). It is also understood that "equivalent diameter" refers to the approximate diameter of a comparable circular cylinder having the same volume as that of the pore.

Depending of the absorptive material, the particles of chromogenic absorbent material may have a pore density greater than about 20%, or greater than about 25%, or of about 27% to about 33%, for example. The pores of the particles of chromogenic absorbent material have an equivalent diameter greater than about 20 µm, or of about 20 µm to about 40 µm, or of about 20 µm to about 30 µm.

Cross sections of extruded starch particles were also examined as a comparative example (see also Example 6), and can be seen in Figs. 7A and 7B.

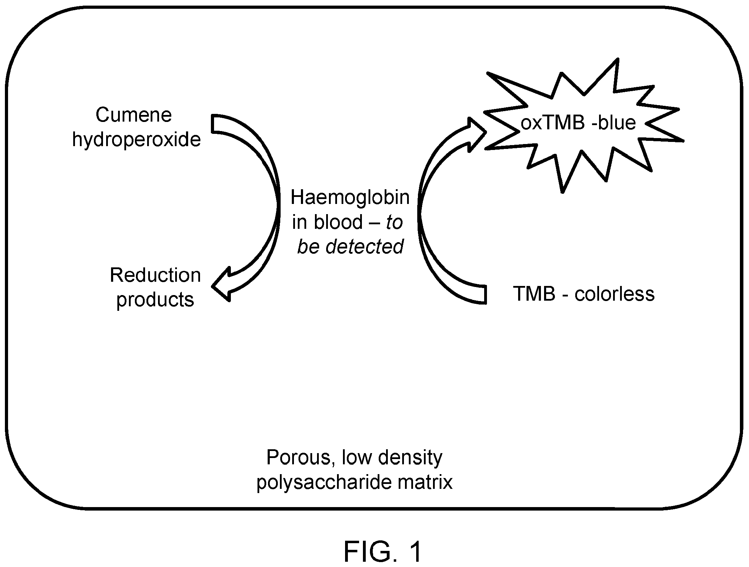

Now referring to Fig. 1, an example of chromogenic absorbent material for detecting blood in animal excretions is described. The substance to be detected (blood) includes haemoglobin which is a pseudoperoxidase. In the absence of blood (i.e., in the absence of peroxidase and/or pseudoperoxidase), the reduction of cumene hydroperoxide (the oxidizing agent) into reduction products and the oxidation of TMB into oxidized TMB (oxTMB) is not catalyzed. When traces of blood are present (i.e., when traces of haemoglobin are present), the reactions are enabled and TMB is oxidized into oxTMB which has a distinctive blue color. The chromogenic absorbent material may be obtained to include a porous polysaccharide matrix having a low density. Thus, the chromogenic absorbent material described is suited for the detection of blood in animal excretions, and therefore for detection of urinary tract diseases for example.

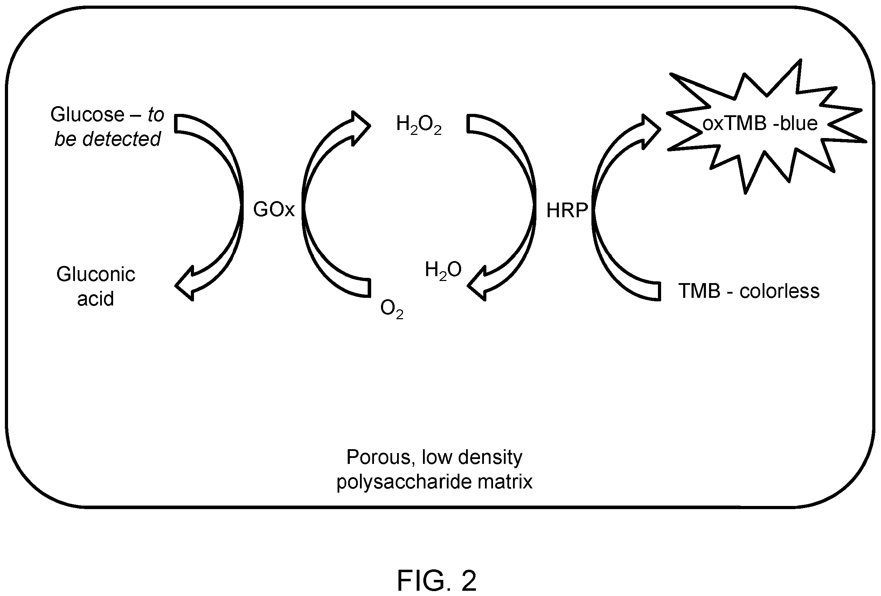

Now referring to Fig. 2, an example of the chromogenic absorbent material for detecting glucose in animal excretions is described. The chromogenic absorbent material used for detecting glucose includes a first catalytic compound (such as glucose oxidase) to generate hydrogen peroxide in situ. In the case of glucose detection, the chromogenic absorbent material further includes a second catalytic compound for catalyzing the oxidation of TMB and the reduction of the hydroperoxide. The second catalytic compound may be horseradish peroxidase. It is understood that other peroxidases or pseudoperoxidases may be used in other implementations. It should also be understood that in the case of glucose detection, the polysaccharide matrix does not include polysaccharides which may react with the first catalytic compound. If such polysaccharides were used, hydrogen peroxide would be generated in situ even without the presence of glucose in the animal excretions, which would lead to false positive test results. For example, when the first catalytic compound is glucose oxidase, the absorptive material does not include starches or modified starches that could react and give false positives.

Still referring to Fig. 2, when glucose is not present in the animal excretions, TMB is not oxidized, as no hydrogen peroxide is generated in situ. When glucose is present in the animal excretions, glucose oxidase oxidizes the glucose into gluconic acid and reduces oxygen into hydrogen peroxide. The horseradish peroxidase then reduces the hydrogen peroxide into water and oxidizes TMB into oxTMB which has a distinctive blue color. The chromogenic absorbent material described in Fig. 2 may be obtained to include a porous polysaccharide matrix having a low density, and is suited for detection of glucose in animal excretions, and therefore for detection of diabetes in animals for example.

In another aspect, a process of manufacturing particles of chromogenic absorbent material is provided. The process includes the steps of:
mixing together a water-absorbing polysaccharide, a second polysaccharide and optionally an superabsorbent polymer, thereby obtaining an absorptive powder mixture;
preparing a chromogenic solution by addition of a chromogenic agent and an oxidizing agent or by addition of the chromogenic agent and a first catalytic compound, into a solution;
combining the chromogenic solution with the absorptive powder mixture so as to obtain solution-impregnated humid particles; and
drying the solution-impregnated humid particles to obtain the chromogenic absorbent material.

It is understood that the step of mixing may not performed in scenarios where the absorptive powder only includes one polysaccharide.

The chromogenic solution includes either the chromogenic agent and the oxidizing agent or the chromogenic agent and a first catalytic compound for generating the oxidizing agent in situ. In the case of chromogenic solutions used for making particles of chromogenic absorbent material for the detection of glucose in animal excretions, the chromogenic solution further includes a second catalytic compound which may include a peroxidase, a pseudoperoxidase, or a mixture thereof.

Optionally, the chromogenic solution may include a buffering agent so as to maintain a pH of the chromogenic solution between 5 and 7. Extreme pH may be avoided.

Optionally, the chromogenic solution may include a colour enhancer, a stabilizer, a metal-scavenger agent or a combination thereof as defined above.

In an optional aspect, the chromogenic solution may be prepared and tailored to the particular absorptive material.

Optionally, the chromogenic solution may be combined with the absorptive material using a low-shear method. For example, the chromogenic solution may be combined with the absorptive material using a one-step granulation in a fluidized bed granulator. For example, the chromogenic solution may also be poured onto the absorptive powder mixture to obtain the solution-impregnated humid particles. In another example, the chromogenic solution may be poured onto the absorptive powder mixture to form the absorptive material, or dripped in the form of discrete drops onto the absorptive powder mixture such that the drops are impregnated with respective amounts of the powder to obtain corresponding discrete solution-impregnated humid particles.

Optionally, the solution-impregnated humid particles may be recovered by filtering the mixture of solution-impregnated humid particles and remaining absorptive powder through a sieve.

The drying step may be performed under vacuum and/or at various temperatures ranging from ambient temperature to about 65°C.

Using low-shear methods as described above allows for the manufacture of particles of chromogenic absorbent material having a lower density, a higher porosity, different morphology, and enhanced absorption properties compared with other types of particles obtained by methods such as extrusion or pressing.

### EXAMPLES

### Example 1

Experiments were performed by preparing particles of chromogenic absorbent material having different compositions and testing the particles when contacted with a blood-containing solution.

Particles of chromogenic absorbent material were prepared by mixing pregelatinized starch (PGS), microcrystalline cellulose (MCC) and sodium polyacrylate as the superabsorbent polymer (SAP), in powder form, thereby obtaining an absorptive powder mixture; pouring the chromogenic solution on the absorptive powder mixture to obtain solution-impregnated humid particles; and drying the solution-impregnated humid particles in an oven at 65 °C to obtain the particles of chromogenic absorbent material. In this case, the particles of chromogenic absorbent material are in the form of granules having a length of between about 0.25 cm and about 0.75 cm.

The chromogenic solution I that was used is detailed in Table 1:

**Table 1**

| **Compound** | **Molar mass (g/mol)** | **Mass or volume** | **Concentration (mmol/L)** |
|---|---|---|---|
| Water (solvent) | - | 50 mL | - |
| Acetone (solvent) | - | 50 mL | - |
| TMB (chromogenic indicator) | 240.34 | 312 mg | 13 |
| CHP (oxidizing agent) | 152.19 | 114 mg | 7.5 |
| 4-lepidine (color enhancer) | 143.19 | 107 mg | 7.5 |
| Polyvinylpyrrolidone (stabilizer) | - | 30 mg | - |
| Ascorbic acid (stabilizer) | 176.12 | 20 mg | 1.15 |

The particles of chromogenic absorbent material were prepared with varying ratios of PGS/MCC and a varying amount of sodium polyacrylate-based SAP, and are numbered as shown in Table 2:

**Table 2**

| | 0 wt.% sodium polyacrylate | 1 wt.% sodium polyacrylate | 2 wt.% sodium polyacrylate | 3 wt.% sodium polyacrylate |
|---|---|---|---|---|
| 35% PGS / 65% MCC | 1 | 2 | 3 | 4 |
| 40%PGS / 60% MCC | 5 | 6 | 7 | 8 |
| 45% PGS / 55% MCC | 9 | 10 | 11 | 12 |
| 55% PGS / 45% MCC | 13 | 14 | 15 | 16 |
| 60% PGS / 40% MCC | 17 | 18 | 19 | 20 |
| 65% PGS / 35% MCC | 21 | 22 | 23 | 24 |

The particles of chromogenic absorbent material shown in Table 2 were placed on a bentonite-based litter and contacted with 5mL of a 0.0215% blood solution or 5 mL of demineralized water which did not contain blood. Particles which were not contacted with any solution were also placed on the litter as a negative control.

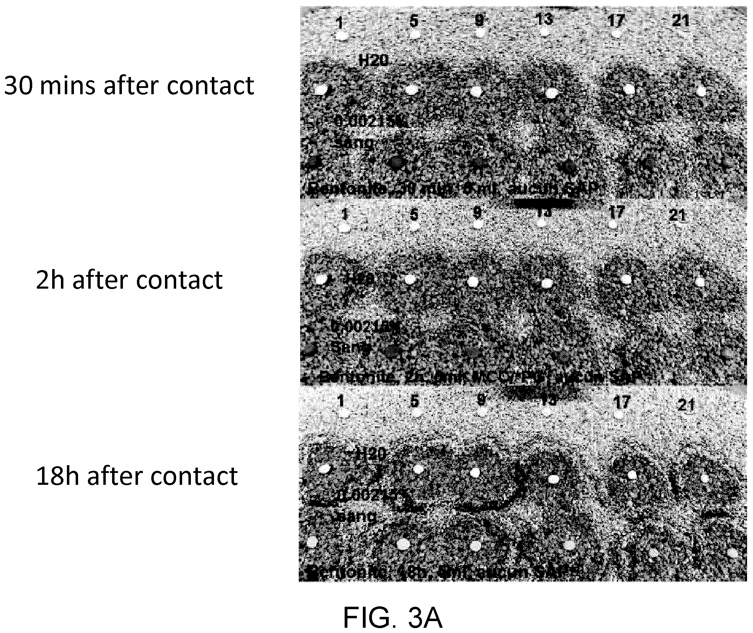

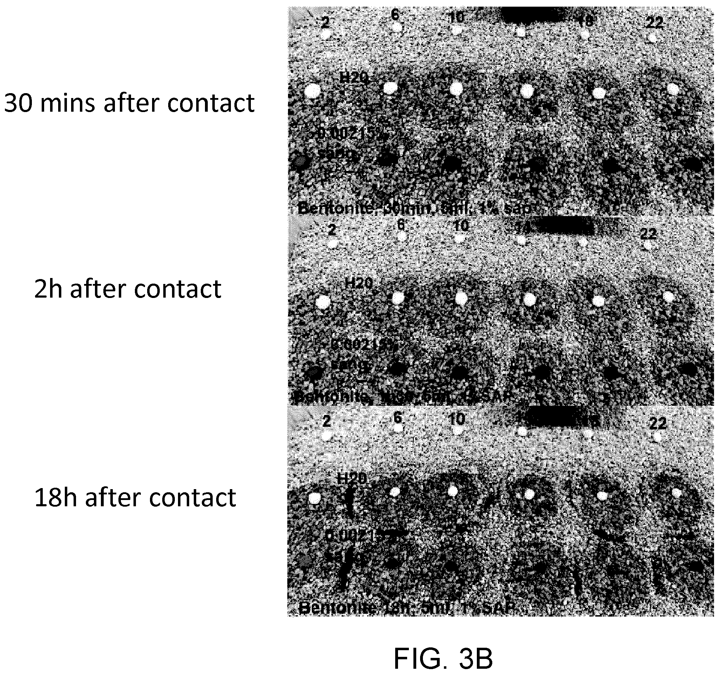

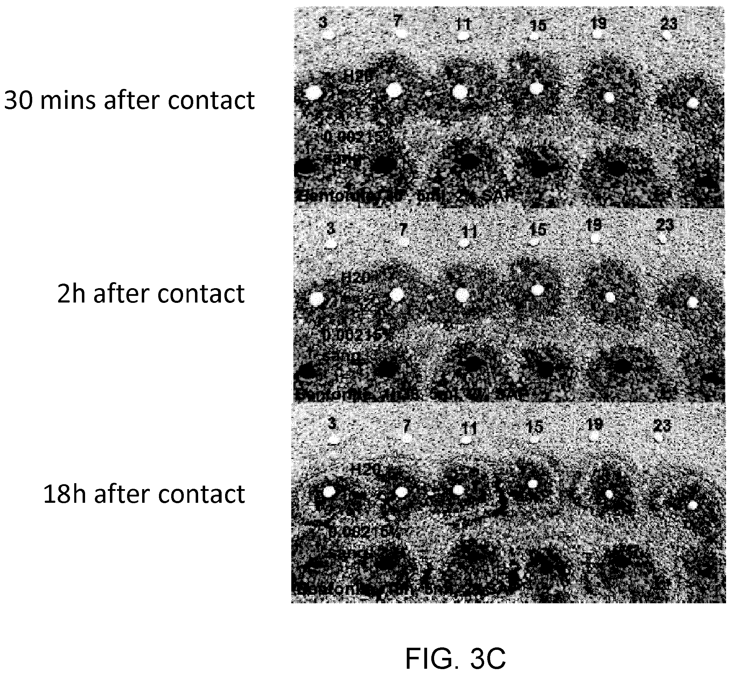

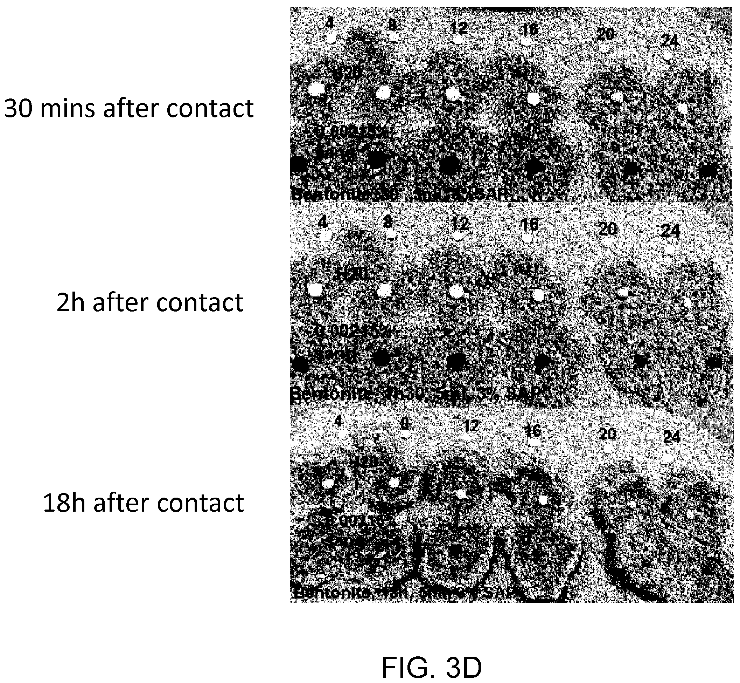

Figs. 3A, 3B, 3C and 3D illustrate samples as numbered in Table 2, and placed on a bentonite-based litter. In each figure, the top picture shows the granules 30 minutes after contact with the solutions, the middle picture shows the granules 2 hours after contact, and the bottom picture shown the granules 18 hours after contact. In each picture of each Figure, the top row of granules is the negative control; the middle row shows granules contacted with 5 mL of demineralized water which did not contain blood; and the bottom row shows granules contacted with 5 mL of a 0.0215% blood solution.

As can be seen in Fig. 3A, granules No. 1, 5, 9, 13, 17 and 21 were contacted with the different solutions (these granules contained 0 wt.% of superabsorbent polymer). The granules contacted with demineralized water did not change color and had the same white color as the negative control granules 30 mins, 2h and 18h after contact. The granules contacted with the blood solution had already turned blue 30 mins after contact. The blue coloration was distinctive. 2h after contact, the blue coloration was still distinctive and present. 18h after contact, the blue coloration had faded and the granules turned off-white or yellow. The blue coloration was present and distinctive for about 8 hours before fading.

As can be seen in Fig. 3B, granules No. 2, 6, 10, 14, 18 and 22 were contacted with the different solutions (these granules contained about 1 wt.% of superabsorbent polymer). The granules contacted with demineralized water did not change color and had the same white color as the negative control granules 30 mins, 2h and 18h after contact. The granules contacted with the blood solution had already turned blue 30 mins after contact. The blue coloration was distinctive. 2h after contact, the blue coloration was still distinctive and present. 18h after contact, the blue coloration was still distinctive and present. The addition of 1 wt.% SAP had a positive effect on the retention of blue coloration in the granules after contact with a blood solution.

As can be seen in Fig. 3C, granules No. 3, 7, 11, 15, 19 and 24 were contacted with the different solutions (these granules contained about 2 wt.% of superabsorbent polymer). The same results as the ones observed and illustrated in Fig. 3B were obtained.

As can be seen in Fig. 3D, granules No. 4, 8, 12, 16, 20 and 25 were contacted with the different solutions (these granules contained about 3 wt.% of superabsorbent polymer). The same results as the ones observed and illustrated in Figs. 3B and 3C were obtained.

### Example 2

Experiments were performed by preparing particles of chromogenic absorbent material using different polysaccharides and mixtures thereof, and testing said particles when contacted with a blood-containing solution. The polysaccharides used in this Example were pregelatinized starch (PGS), microcrystalline cellulose (MCC) and carboxymethylcellulose (CMC).

The particles were prepared as described in Example 1. No superabsorbent polymer was used in this Example and the mixing step was not performed when only one polysaccharide was used. The same chromogenic solution I as described in Example 1 was also used.

Particles of chromogenic absorbent material were prepared using various polysaccharides and mixtures thereof, and are numbered as shown in Table 3.

**Table 3**

| **Polysaccharide or polysaccharide mixture** | **Sample number** |
|---|---|
| 50% PGS / 50% MCC | 25 |
| 100 % CMC | 26 |
| 100 % PGS | 27 |

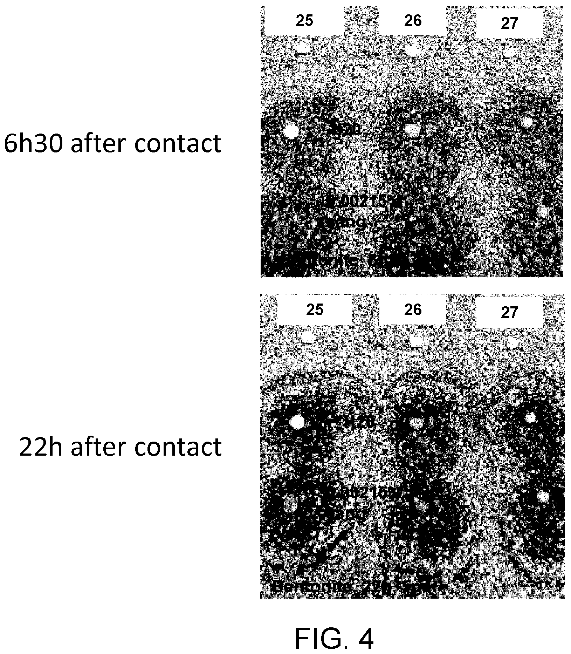

Fig. 4 shows the granules 6h30 and 22h after contact with 5mL of demineralized water which did not contain blood (middle row) or 5mL of a 0.0215% blood solution (bottom row). The top row is the negative control showing granules which were not contacted with either solution. A deep blue coloration rapidly appeared a few minutes after contact with the blood-containing solution (not shown). The granules contacted with demineralized water stayed substantially white or became slightly yellow. After 6h30, samples No. 25 and 26 retained the deep blue coloration, while the blue coloration of sample No. 27 was lighter. After 22h, sample No. 25 retained the deep blue coloration, sample No. 26 had a light blue coloration, and the coloration of sample No. 27 had substantially faded.

It is to be noted that all the samples prepared enable the detection of blood. Using 50% PGS / 50% MCC as the absorptive material enabled the blue coloration to be retained for a longer period when compared with 100% CMC and 100% PGS granules.

### Example 3

Experiments have been performed by preparing particles of chromogenic absorbent material using a mixture of 50% microcrystalline cellulose (MCC) and 50% carboxymethyl cellulose (CMC) as the absorptive material, and different chromogenic solutions. Said particles were contacted with glucose-containing solutions.

The composition of the chromogenic solution II is detailed in Table 4.

**Table 4**

| **Solvents and compounds** | **Mass or volume** |
|---|---|
| Water (solvent) | 50 mL |
| Acetone (solvent) | 50 mL |
| TMB (chromogenic indicator) | 312 mg |
| Glucose oxidase (first catalytic compound) | 6 mg |
| Horseradish peroxidase (second catalytic compound) | 5 mg |

Chromogenic solution II shown in Table 4 was diluted at ratios of 1:2 and 1:10 to obtain chromogenic solutions III (1:2 dilution) and IV (1:10 dilution).

Particles of chromogenic absorbent material were prepared by mixing carboxymethyl cellulose (CMC) and microcrystalline cellulose (MCC), thereby obtaining an absorptive powder mixture; pouring chromogenic solution II, III or IV on the absorptive powder mixture to obtain solution-impregnated humid particles; and drying the solution-impregnated humid particles in an oven at 65 °C to obtain the particles of chromogenic absorbent material. In this case, the particles of chromogenic absorbent material were obtained in the form of granules.

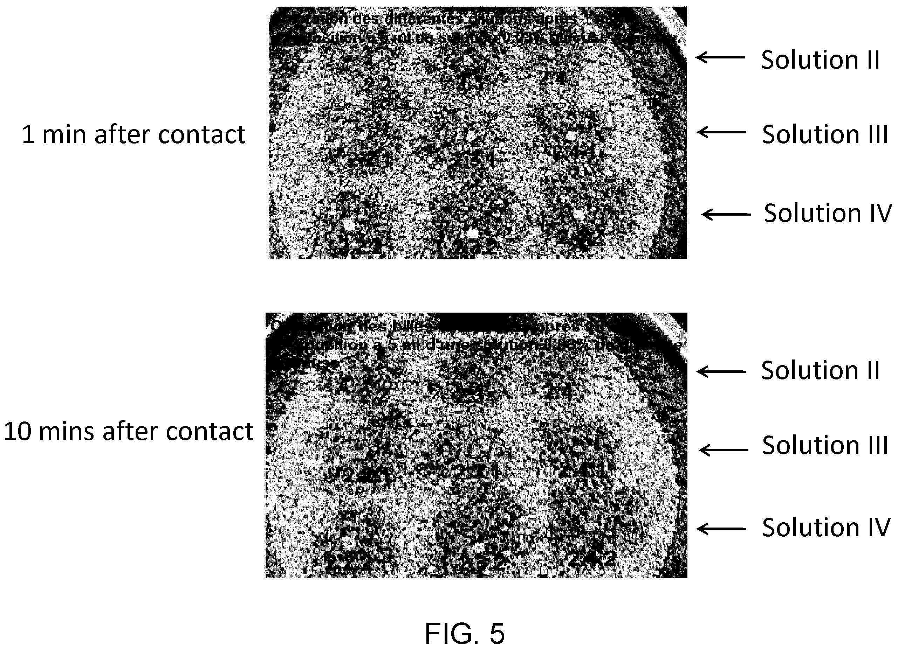

Figure 5 shows particles of chromogenic absorbent material 1 minute (top picture) and 10 minutes (bottom picture) after contact with a solution containing 0.03% of glucose. In each picture, the top row corresponds to chromogenic absorbent material made with chromogenic solution II, the middle row corresponds to chromogenic absorbent material made with chromogenic solution III, and the bottom row corresponds to chromogenic absorbent material made with chromogenic solution IV. As can be seen, when the more concentrated solution II was used, the blue coloration is deeper and appears within 1 minute of contact. When the lower concentration solution IV is used, the deep blue coloration appeared within 10 minutes of contact.

### Example 4

Experiments were also performed by measuring the free swelling capacity (FSC) of particles of chromogenic absorbent material. The particles of chromogenic absorbent material were prepared as described in Example 1 using PGS, Xanthan or guar as the water-absorbing polysaccharide, and MCC. The measurements were performed by soaking the samples in water for 30 minutes and draining the water remaining at the surface for 10 minutes. The values obtained were compared with the FSC values of particles obtained by extrusion or pressing. The results are detailed in Table 5.

**Table 5**

| **Particle type** | **FSC %** |
|---|---|
| Extruded starch granule without gas injection (comparative) | 190 |
| Extruded starch granule with gas injection (comparative) | 200 |
| Pressed paper pulp pellet (comparative) | 500 |
| | |
| 50% PGS / 50% MCC granule (sample No. 25 of Example 2) | 1080 |
| 50% Xanthan / 50% MCC granule | 3360 |
| 50% guar gum / 50% MCC granule | 2030 |

The particles of chromogenic absorbent material made from PGS/MCC, xanthan/MCC and guar gum/MCC all exhibit high FSC values. This is indicative of a very high porosity and surprisingly high absorption properties when compared with the extruded starch granules and pressed paper pulp pellets known in the art.

### Example 5

Experiments have also been performed by measuring the density of particles of chromogenic absorbent material. The particles of chromogenic absorbent material were prepared as described in Example 1 using PGS, Xanthan or guar as the water-absorbing polysaccharide, and MCC. The values obtained were compared with the density values of particles known in the art and obtained by extrusion or pressing. The results are detailed in Table 6.

**Table 6**

| **Particle type** | **Density (g/cm³)** |
|---|---|
| Extruded starch granule without gas injection (comparative) | 0.60 |
| Extruded starch granule with gas injection (comparative) | 0.48 |
| Pressed paper pulp pellet (comparative) | 0.40 |
| | |
| 50% PGS / 50% MCC granule (sample No. 25 of Example 2) | 0.33 |
| 50% Xanthan / 50% MCC granule | 0.37 |
| 50% guar gum / 50% MCC granule | 0.26 |

The particles of chromogenic absorbent material made from PGS/MCC, xanthan/MCC and guar gum/MCC exhibit lower density values when compared with the extruded starch granules and pressed paper pulp pellets known in the art.

### Example 6

Experiments have been performed to obtain scanning electron micrographs of cross sections of particles of extruded starch with or without injected gas during extrusion (Figures 7A and 7B, comparative) and of a cross section of a particle of chromogenic absorbent material corresponding to sample 25 as shown in Example 2 (Figure 7C). The images obtained were analyzed to determine the pore density and the equivalent diameter of the pores. Prior to imaging, the respective particles were first hardened by freezing in liquid nitrogen and cut in the frozen state. The scanning electron microscope used was a MEB JEOL JSM-5900LV™ (low vacuum).

The pore density and equivalent diameter measurements were performed by using the Nikon NIS-Elements D™ image analysis software. The results are detailed in Table 7.

**Table 7**

| **Particle type** | **Pore density (%)** | **Equivalent diameter (µm)** |
|---|---|---|
| Extruded starch granule without gas injection (comparative) | 7.6 | 7.8 |
| Extruded starch granule with gas injection (comparative) | 10.8 | 11.5 |
| 50% PGS / 50% MCC granule (sample No. 25 of Example 2) | 29.5 | 25.3 |

The particles of corresponding to sample No. 25 of Example 2 have a higher pore density and equivalent pore diameter than the particles of extruded starch (made with or without gas injection during high shear extrusion).

### Example 7

Experiments have been performed on sample No. 25 of Example 2 to measure the total porosity and effective porosity of particles of chromogenic absorbent material. Comparative measurements were also performed on extruded starch granules (with or without injected gas during high shear extrusion). The porosity measurements were performed as follows.

200 mL of particles were placed in a container. The particles were weighed (mass m). Acetone was added to soak the particles and completely cover the particles with solvent. The volume of solvent required to cover all the particles was measured (Vc). The soaked particles were removed from the container and the volume of remaining solvent was measured (Vr). The volume of liquid absorbed by the chromogenic absorbent particles (Va = Vc-Vr) was calculated. The total porosity is then obtained by calculating the ratio of the volume of added liquid (Vc) to the volume of particles (V), and the effective porosity is calculated using Equation 2 detailed above. The results are summarized in Table 8.

**Table 8**

| **Particle type** | **Mass of particles (g)** | **Vc (mL)** | **Va (mL)** | **Total porosity (%)** | **Effective porosity (mL/g)** |
|---|---|---|---|---|---|
| Extruded starch granule without gas injection (comparative) | 120 | 104 | 18 | 52% | 0.15 |
| Extruded starch granule with gas injection (comparative) | 96 | 116 | 16 | 58% | 0.167 |
| 50% PGS / 50% MCC granule (sample No. 25 of Example 2) | 66 | 150 | 65 | 75% | 0.985 |

As can be seen, the particles of chromogenic absorbent material made of 50% PGS and 50% MCC have an effective porosity which is substantially higher than extruded starch particles obtained with or without gas injection during high shear extrusion.

## Claims

1. A chromogenic absorbent material for an animal litter, comprising:
an oxidizing agent responsive to peroxidatic/pseudoperoxidatic activity in an animal excretion to provide oxidizing activity;
a chromogenic indicator being chromogenically responsive to the oxidizing activity of the oxidizing agent; and
an absorptive material which is porous, for absorbing the animal excretion, the
absorptive material comprising a water-absorbing polysaccharide,
wherein the chromogenic absorbent material is a porous material having an effective porosity of about 0.5 mL/g to about 2.0 mL/g,
wherein the effective porosity is measured by placing a known mass (m) of chromogenic absorbent particles into a container, covering it with a liquid, measuring the volume of liquid needed to cover the particles (Vc), removing the soaked particles from the container, measuring the liquid remaining in the container (Vr), calculating the volume of liquid absorbed in the particles (Va = Vc-Vr) and calculating the effective porosity as Va/m,
wherein the chromogenic absorbent material has a free swelling capacity greater than about 900%,
wherein the free swelling capacity is measured by soaking the material in water for 30 minutes, draining it for 10 minutes and weighing the material after the liquid has been absorbed, and
wherein the chromogenic absorbent material is provided with pores having an equivalent diameter greater than about 20 µm or an equivalent diameter of about 20 µm to about 40 µm, preferably about 20 µm to about 30 µm, wherein the equivalent diameter is measured by measuring the pore diameters of the cross-section of a sample of the chromogenic absorbent material under a scanning electron microscope.

2. The material of claim 1, wherein the effective porosity is of about 0.8 mL/g to about 1.2 mL/g.

3. The material of claim 1 or 2, wherein the chromogenic absorbent material is provided with pores having an equivalent diameter of about 20 µm to about 40 µm.

4. The material of any one of claims 1 to 3, wherein the chromogenic absorbent material is provided with pores having an equivalent diameter of about 20 µm to about 30 µm.

5. The material of any one of claims 1 to 4, having a free swelling capacity greater than about 1000%.

6. The material of any one of claims 1 to 5, wherein the water-absorbing polysaccharide comprises a starch, a modified starch, a cellulose derivative or a gelling polysaccharide, or a mixture thereof.

7. The material of claim 6, wherein the water-absorbing polysaccharide comprises pregelatinized starch.

8. The material of claim 6, wherein the cellulose derivative comprises a cellulose ester or a cellulose ether, or a mixture thereof.

9. The material of claim 6, wherein the cellulose derivative comprises carboxymethyl cellulose (CMC).

10. The material of claim 6, wherein the gelling polysaccharide comprises agar-agar, guar or xanthan, or a mixture thereof.

11. The material of any one of claims 1 to 9, wherein the oxidizing agent comprises a hydroperoxide or a hydroperoxide precursor, or a combination thereof.

12. The material of claim 11, wherein the hydroperoxide comprises hydrogen peroxide, cumene hydroperoxide or diisopropylbenzene dihydroperoxide, or a combination thereof.

13. The material of any one of claims 1 to 12, wherein the oxidizing agent and the chromogenic indicator are distributed within the absorptive material.

14. The material of any one of claims 1 to 13, wherein the chromogenic indicator comprises a benzidine-type compound.

15. The material of claim 14, wherein the benzidine-type compound comprises 3,3',5,5'-tetramethylbenzidine.

## Patentansprüche

1. Chromogenes absorbierendes Material für eine Tierstreu, umfassend:
ein Oxidationsmittel, das auf peroxidatische/pseudoperoxidatische Aktivität in einer tierischen Ausscheidung reagiert, um Oxidationsaktivität bereitzustellen;
einen chromogenen Indikator, der auf die Oxidationsaktivität des Oxidationsmittels chromogen reagiert; und
ein absorptionsfähiges Material, das porös ist, um die tierische Ausscheidung zu absorbieren, wobei das absorptionsfähige Material ein wasserabsorbierendes Polysaccharid umfasst,
wobei es sich bei dem chromogenen absorbierenden Material um ein poröses Material handelt, das eine effektive Porosität von etwa 0,5 mL/g bis etwa 2,0 mL/g aufweist,
wobei die effektive Porosität durch Geben einer bekannten Masse (m) von chromogenen absorbierenden Partikeln in einen Behälter, Bedecken derselben mit einer Flüssigkeit, Messen des Flüssigkeitsvolumens, das erforderlich ist, um die Partikel zu bedecken (Vc), Entfernen der getränkten Partikel aus dem Behälter, Messen der im Behälter verbleibenden Flüssigkeit (Vr), Berechnen des in den Partikeln absorbierten Flüssigkeitsvolumens (Va = Vc-Vr) und Berechnen der effektiven Porosität als Va/m gemessen wird,
wobei das chromogene absorbierende Material ein freies Quellvermögen von mehr als etwa 900 % aufweist,
wobei das freie Quellvermögen durch 30-minütiges Tränken des Materials in Wasser, 10-minütiges Abtropfenlassen desselben, und Wiegen des Materials, nachdem die Flüssigkeit absorbiert wurde, gemessen wird, und
wobei das chromogene absorbierende Material mit Poren versehen ist, die einen äquivalenten Durchmesser von mehr als etwa 20 µm, oder einen äquivalenten Durchmesser von etwa 20 µm bis etwa 40 µm, vorzugsweise etwa 20 µm bis etwa 30 µm aufweisen, wobei der äquivalente Durchmesser durch Messen der Porendurchmesser des Querschnitts einer Probe des chromogenen absorbierenden Materials unter einem Rasterelektronenmikroskop gemessen wird.

2. Material nach Anspruch 1, wobei die effektive Porosität etwa 0,8 mL/g bis etwa 1,2 mL/g beträgt.

3. Material nach Anspruch 1 oder 2, wobei das chromogene absorbierende Material mit Poren versehen ist, die einen äquivalenten Durchmesser von etwa 20 µm bis etwa 40 µm aufweisen.

4. Material nach einem der Ansprüche 1 bis 3, wobei das chromogene absorbierende Material mit Poren versehen ist, die einen äquivalenten Durchmesser von etwa 20 µm bis etwa 30 µm aufweisen.

5. Material nach einem der Ansprüche 1 bis 4, das ein freies Quellvermögen von mehr als etwa 1000 % aufweist.

6. Material nach einem der Ansprüche 1 bis 5, wobei das wasserabsorbierende Polysaccharid eine Stärke, eine modifizierte Stärke, ein Cellulosederivat oder ein gelbildendes Polysaccharid, oder ein Gemisch davon umfasst.

7. Material nach Anspruch 6, wobei das wasserabsorbierende Polysaccharid vorgelierte Stärke umfasst.

8. Material nach Anspruch 6, wobei das Cellulosederivat einen Celluloseester oder einen Celluloseether, oder ein Gemisch davon umfasst.

9. Material nach Anspruch 6, wobei das Cellulosederivat Carboxymethylcellulose (CMC) umfasst.

10. Material nach Anspruch 6, wobei das gelbildende Polysaccharid Agar-Agar, Guar oder Xanthan, oder ein Gemisch davon umfasst.

11. Material nach einem der Ansprüche 1 bis 9, wobei das Oxidationsmittel ein Hydroperoxid oder einen Hydroperoxid-Vorläufer, oder eine Kombination davon umfasst.

12. Material nach Anspruch 11, wobei das Hydroperoxid Wasserstoffperoxid, Cumolhydroperoxid oder Diisopropylbenzoldihydroperoxid, oder eine Kombination davon umfasst.

13. Material nach einem der Ansprüche 1 bis 12, wobei das Oxidationsmittel und der chromogene Indikator im absorptionsfähigen Material verteilt sind.

14. Material nach einem der Ansprüche 1 bis 13, wobei der chromogene Indikator eine Verbindung vom Benzidin-Typ umfasst.

15. Material nach Anspruch 14, wobei die Verbindung vom Benzidin-Typ 3,3',5,5'-Tetramethylbenzidin umfasst.

## Revendications

1. Matériau absorbant chromogène pour une litière animale, comprenant :
un agent oxydant réactif à une activité peroxydase/pseudoperoxydase dans une excrétion animale pour fournir une activité oxydante ;
un indicateur chromogène réactif de manière chromogène à l'activité oxydante de l'agent oxydant ; et
un matériau absorbant qui est poreux, pour absorber l'excrétion animale, le matériau absorbant comprenant un polysaccharide hydroabsorbant,
dans lequel le matériau absorbant chromogène est un matériau poreux présentant une porosité effective d'environ 0,5 ml/g à environ 2,0 ml/g,
dans lequel la porosité effective est mesurée en plaçant une masse connue (m) de particules absorbantes chromogènes dans un récipient, en les recouvrant avec un liquide, en mesurant le volume de liquide nécessaire pour recouvrir les particules (Vc), en retirant les particules imbibées du récipient, en mesurant le liquide restant dans le récipient (Vr), en calculant le volume de liquide absorbé dans les particules (Va= Vc - Vr) et en calculant la porosité effective par Va/m,
dans lequel le matériau absorbant chromogène présente une capacité de gonflement libre supérieure à environ 900 %,
dans lequel la capacité de gonflement libre est mesurée en trempant le matériau dans de l'eau pendant 30 minutes, en la drainant pendant 10 minutes et en pesant le matériau après que le liquide a été absorbé, et
dans lequel le matériau absorbant chromogène est pourvu de pores présentant un diamètre équivalent supérieur à environ 20 µm ou un diamètre équivalent d'environ 20 µm à environ 40 µm, de préférence d'environ 20 µm à environ 30 µm,
dans lequel le diamètre équivalent est mesuré en mesurant les diamètres de pore de la section transversale d'un échantillon du matériau absorbant chromogène sous un microscope électronique à balayage.

2. Matériau selon la revendication 1, dans lequel la porosité effective est d'environ 0,8 ml/g à environ 1,2 ml/g.

3. Matériau selon la revendication 1 ou 2, dans lequel le matériau absorbant chromogène est pourvu de pores présentant un diamètre équivalent d'environ 20 µm à environ 40 µm.

4. Matériau selon l'une quelconque des revendications 1 à 3, dans lequel le matériau absorbant chromogène est pourvu de pores présentant un diamètre équivalent d'environ 20 µm à environ 30 µm.

5. Matériau selon l'une quelconque des revendications 1 à 4, présentant une capacité de gonflement libre supérieure à environ 1 000 %.

6. Matériau selon l'une quelconque des revendications 1 à 5, dans lequel le polysaccharide hydroabsorbant comprend un amidon, un amidon modifié, un dérivé de cellulose ou un polysaccharide gélifiant, ou un mélange de ceux-ci.

7. Matériau selon la revendication 6, dans lequel le polysaccharide hydroabsorbant comprend un amidon prégélatinisé.

8. Matériau selon la revendication 6, dans lequel le dérivé de cellulose comprend un ester de cellulose ou un éther de cellulose, ou un mélange de ceux-ci.

9. Matériau selon la revendication 6, dans lequel le dérivé de cellulose comprend une carboxyméthylcellulose (CMC).

10. Matériau selon la revendication 6, dans lequel le polysaccharide gélifiant comprend un agar-agar, un guar ou un xanthane, ou un mélange de ceux-ci.

11. Matériau selon l'une quelconque des revendications 1 à 9, dans lequel l'agent oxydant comprend un hydroperoxyde ou un précurseur d'hydroperoxyde, ou une combinaison de ceux-ci.

12. Matériau selon la revendication 11, dans lequel l'hydroperoxyde comprend le peroxyde d'hydrogène, l'hydroperoxyde de cumène ou le dihydroperoxyde de diisopropylbenzène, ou une combinaison de ceux-ci.

13. Matériau selon l'une quelconque des revendications 1 à 12, dans lequel l'agent oxydant et l'indicateur chromogène sont répartis dans le matériau absorbant.

14. Matériau selon l'une quelconque des revendications 1 à 13, dans lequel l'indicateur chromogène comprend un composé de type benzidine.

15. Matériau selon la revendication 14, dans lequel le composé de type benzidine comprend la 3,3',5,5'-tétraméthylbenzidine.
